Europäisches Patentamt

European Patent Office

Office européen des brevets

(19)

(11) Veröffentlichungsnummer : **0 188 783**
**B1**

(12)

# EUROPÄISCHE PATENTSCHRIFT

(45) Veröffentlichungstag der Patentschrift :
25.05.88

(51) Int. Cl.⁴ : **G 21 K 1/04**, **H 04 N 5/32**, **A 61 B 6/06**

(21) Anmeldenummer : 85116309.7

(22) Anmeldetag : 20.12.85

(54) Röntgendiagnostikeinrichtung mit halbtransparenter Blende.

(30) Priorität : 11.01.85 DE 3500812

(43) Veröffentlichungstag der Anmeldung :
30.07.86 Patentblatt 86/31

(45) Bekanntmachung des Hinweises auf die Patenterteilung : 25.05.88 Patentblatt 88/21

(84) Benannte Vertragsstaaten :
BE DE FR GB IT

(56) Entgegenhaltungen :
EP-A- 0 083 465
EP-A- 0 114 369
EP-A- 0 158 382
DE-A- 1 800 879
DE-A- 2 053 089
US-A- 2 943 205
US-A- 3 912 936

(73) Patentinhaber : Siemens Aktiengesellschaft Berlin
und München
Wittelsbacherplatz 2
D-8000 München 2 (DE)

(72) Erfinder : Hüttenrauch, Gerd
Sudetenstrassse 14
D-8525 Uttenreuth (DE)
Erfinder : Jann, Wolfango
Hutweide 34
D-8520 Buckenhof (DE)
Erfinder : Kranvogel, Feliks
Fliederweg 31
D-8524 Neunkirchen (DE)

EP 0 188 783 B1

Jouve, 18, rue St-Denis, 75001 Paris, France

**Beschreibung**

Die Erfindung betrifft eine Röntgendiagnostikeinrichtung mit einer Röntgenröhre, einer zumindest bereichsweise halbtransparenten Blende, die einen in seiner Breite veränderbaren, die Strahlung durchlassenden Schlitz freigibt, mit einem Röntgenbildverstärker und einer daran angekoppelten Fernsehkamera zur Erzeugung von Videosignalen, die zur Wiedergabe der Videosignale mit einem Monitor verbunden ist.

Aus der DE-AS 1 800 879 ist eine Primärstrahlenblende für Röntgenuntersuchungsgeräte bekannt, bei der eine aus zwei Blendenplatten bestehende halbtransparente Blende die seitlichen Strahlungen im Röntgenstrahlenbündel schwächt, die sonst insbesondere bei Extremitäten ungeschwächt auf den Röntgenbildverstärker-Eingangsleuchtschirm auffallen und somit helle Bereiche erzeugen, die die Detailerkennbarkeit im eigentlichen Betrachtungsbereich beeinträchtigen. Durch die Verwendung der halbtransparenten Blendenplatten werden diese überstrahlten seitlichen Bereiche abgeschwächt, so daß sich die Sichtbarkeit im interessierenden Bereich erhöht, wobei aber kontraststarke Gegenstände, beispielsweise Operationsbestecke, die seitlich herangeführt werden, noch deutlich sichtbar sind. Zur Anpassung der Ausrichtung des Untersuchungsobjektes im Hinblick auf die Röntgendiagnostikeinrichtung sind die Blendenplatten auf einer Blendenscheibe drehbar angeordnet. Zur manuellen Einstellung der Breite des ungeschwächten Strahlenganges lassen sich beide Blendenplatten aufeinander zu bewegen, bis beispielsweise sämtliche überstrahlten Bereiche verschwunden sind. Bei nicht geradlinigen Konturen, wie sie normalerweise vorkommen, läßt sich mit diesen geradlinigen Stirnflächen der Blendenplatten keine Konturenanpassung erreichen. Entweder wird ein großer Anteil des zu untersuchenden Objektes mit abgeschwächt oder aber es werden noch große Teile des Fernsehbildes überstrahlt, so daß auch weiterhin die Sichtbarkeit von Details reduziert ist.

Es ist weiterhin aus der DE-OS 29 05 202 ein Lichtschaukasten bekannt, bei dem zur Betrachtung von transparenten, rechteckigen Bildern die Bildauflage durch eine Vielzahl parallelliegender Abdeckstreifen geringer Breite abdunkelbar ist. Durch Auflegen von Bildern auf den Rand des Lichtschaukastens wird deren Größe automatisch erfaßt. Anschließend werden nur diejenigen Streifen, die das Bild verdecken, durch einen Motor so lange bewegt, bis der Film auch an seiner Unterkante ausgeleuchtet ist. Gleichzeitig können mehrere Filme nur dann ausgeleuchtet werden, wenn sie vertikal gemessen gleich groß sind. Das bedeutet aber auch, daß eine Anpassung an unregelmäßige Konturen durch die Lamellen dieses Lichtschaukastens nicht ermöglicht wird.

Die Erfindung geht von der Aufgabe aus, eine Röntgendiagnostikeinrichtung der eingangs genannten Art zu schaffen, bei der sich die halbtransparente Blende möglichst genau den Konturen des zu untersuchenden Objektes anpaßt, so daß gar keine oder nur kleine Bereiche des Untersuchungsobjektes verdeckt oder nur kleine Bereiche in der Nähe des Objektes nicht abgedeckt sind.

Die Aufgabe wird erfindungsgemäß dadurch gelöst, daß die Blende aus mehreren einzelnen Lamellen besteht, die zu beiden Seiten des Schlitzes parallel aneinander anliegen und individuell in Abhängigkeit von der Größe des zu betrachtenden Gegenstandes in Längsrichtung verstellbar sind, und daß an der Fernsehkamera eine Auswerteschaltung angeschlossen ist, die ein aus dem Videosignal gewonnenes Steuersignal einer Stellvorrichtung für die einzelnen Lamellen zuführt. Dadurch ist es möglich, daß automatisch die Lamellen der Blende aufeinander zu bewegt werden, bis daß jede einzelne Lamelle die äußeren Konturen des Untersuchungsobjektes berührt.

Ein einfacher Aufbau der Auswerteschaltung ergibt sich, wenn die Längsrichtung der Lamellen senkrecht zur Mittellinie des Schlitzes weist. Eine bessere Anpassung der Lamellen an die Kontur des Untersuchungsobjektes kann erreicht werden, wenn die Längsrichtung der Lamellen mit der Mittellinie des Schlitzes einen Winkel zwischen 20° und 90° bildet. Es hat sich als vorteilhaft erwiesen, wenn der Winkel zwischen Längsrichtung der Lamellen und der Mittellinie des Schlitzes 45° beträgt. Eine einfache Steuerung der Lamellen ergibt sich, wenn die einzelnen Lamellen durch je einen Motor verschoben werden. Der mechanische Aufbau läßt sich vereinfachen, wenn für jede Seite der Blende ein Motor, der eine Walze antreibt, und wenigstens ein Kuppelmagnet vorgesehen sind, die von der Auswerteschaltung gesteuert werden, und wenn die Lamellen durch den Kuppelmagneten mit der Walze kuppelbar sind. Ein sicheres Verstellen der Lamellen wird erreicht, wenn die Lamellen wenigstens bereichsweise als Zahnstange ausgebildet sind und wenn die Walze als Zahnwalze ausgebildet ist. Der mechanische Aufwand kann weiterhin reduziert werden, wenn ein Kuppelmagnet vorgesehen ist, der mechanisch verschoben wird und die Lamellen einzeln mit der Walze des Motors kuppelt. Eine parallele Ansteuerung der Lamellen kann erreicht werden, wenn jeder Lamelle ein Kuppelmagnet zugeordnet ist, der von der Auswerteschaltung getrennt angesteuert wird. Die Auswerteschaltung vereinfacht sich weiterhin, wenn die Kuppelmagnete einzeln nacheinander angesteuert werden. Ein schnelles Verstellen der Lamellen kann dadurch erreicht werden, wenn die Kuppelmagnete alternierend im Multiplexbetrieb durch die Auswerteschaltung angesteuert werden. Der elektronische Aufwand läßt sich vereinfachen, wenn bei einer Röntgendiagnostikeinrichtung, bei der die Blende auf einem drehbaren Blendenring angeordnet ist, die Fernsehkamera und die Blende in ihrer Drehung miteinander derart gekoppelt sind, daß die Längsrichtung der Lamellen immer in Zeilenrichtung der Fernsehkette liegt.

Ein vorteilhafter Aufbau der Auswerteschaltung ergibt sich, wenn sie eine Schaltung zur Lagebestimmung der Lamellen innerhalb des Videobildes aufweist, der die Taktimpulse der Fernsehkamera und Steuersignale zugeführt werden, die die jeweils angesteuerte Lamelle kennzeichnen, wenn das Videosignal einer Anpaßstufe zugeführt ist, wenn die Anpaßstufe und die Schaltung mit einer Torschaltung verbunden sind, an der ein Spitzenwert-Detektor angeschlossen ist, wenn das Ausgangssignal des Detektors einer Vergleichsstufe zugeführt wird, die es mit einem einstellbaren Schwellenwert vergleicht, und wenn die Vergleichsstufe die Kuppelmagnete steuert. Eine optimale Steuerung der Lamellen wird erreicht, wenn der Schwellenwert organabhängig auswählbar ist.

Die Erfindung ist nachfolgend anhand eines in der Zeichnung dargestellten Ausführungsbeispieles näher erläutert. Es zeigen :

Fig. 1 eine Röntgendiagnostikeinrichtung mit einer Primärstrahlenblende,

Fig. 2 den schematischen Aufbau einer erfindungsgemäßen Blende, und

Fig. 3 ein Blockschaltbild des erfindungswesentlichen Teiles der in Figur 2 dargestellten Auswertelogik.

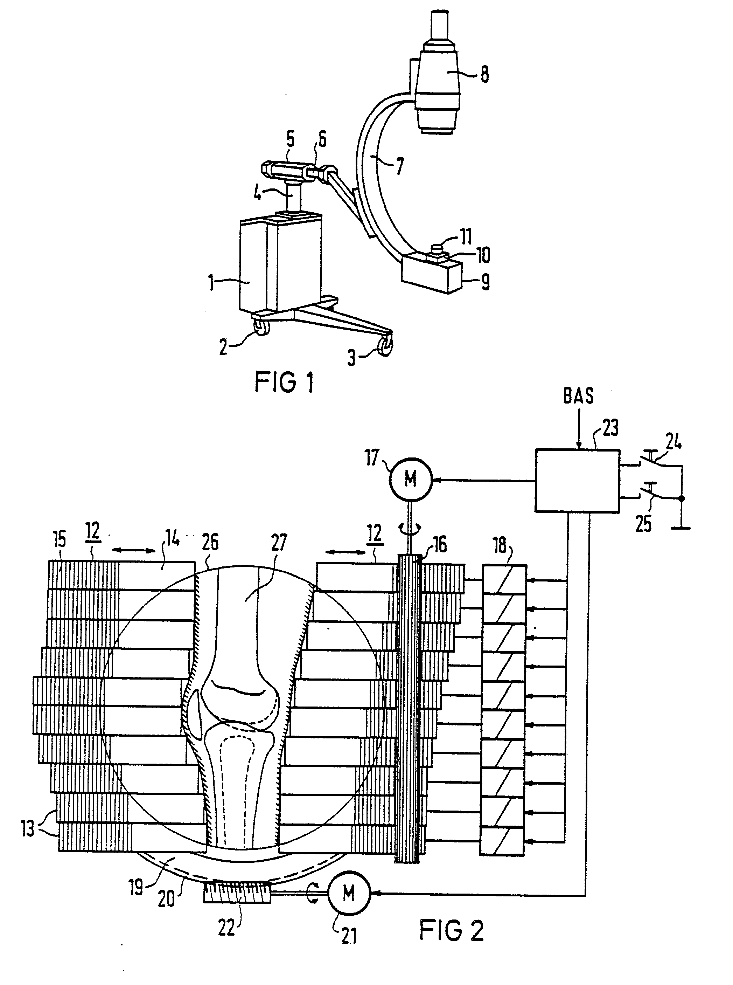

In der Figur 1 ist eine fahrbare, mit Rädern 2 und 3 versehene Röntgendiagnostikeinrichtung 1 dargestellt, die vielfach in der Chirurgie Verwendung findet. Sie weist einen in der Höhe verstellbaren Vertikalträger 4 mit einer Muffe 5 auf, in der ein Horizontalträger 6 verschiebbar gelagert ist. An dem Ende des Horizontalträgers 6 ist ein C-Bogen 7 verschiebbar angeordnet, an dessen einem Ende das Gehäuse 8 für einen Röntgenbildverstärker und eine Fernsehkamera und an seinem anderen Ende das Röntgenröhrengehäuse 9 angebracht ist. An dem Röntgenröhrengehäuse 9 ist eine Primärstrahlenblende 10 angeordnet, an der ein Tubus 11 befestigt ist.

In der Figur 2 ist der schematische Aufbau einer in der Primärstrahlenblende 10 angeordneten, halbtransparenten Blende 12 dargestellt. Sie besteht aus einer Vielzahl parallel nebeneinander angeordneter, streifenförmiger Lamellen 13, die in ihrem aufeinander zu gerichteten Bereich 14 einen Absorptionswert aufweisen, der einer 0,4 mm bis 3,5 mm starken Eisenplatte entspricht. In ihrem hinteren Bereich sind die Lamellen 13 als Zahnstange 15 ausgebildet, in die beidseitig von Motoren 17 (nur einer dargestellt) getriebene Zahnwalzen 16 eingreifen. Jeder Zahnstange 15 der Lamellen 13 ist ein Kuppelmagnet 18 zugeordnet, der bei Betätigung die Zahnstangen 15 gegen die motorgetriebenen Zahnwalzen 16 drückt und den Kraftschluß herstellt.

Diese Anordnung ist mit einem Blendenring 19 verbunden, der auf seinem äußeren Rand Zahnsegmente 20 aufweist, in die eine von einem Motor 21 getriebene Schnecke 22 eingreift so daß die gesamte Blende 12 um ihren Mittelpunkt gedreht werden kann. Eine Auswerteschaltung 23, der das Videosignal BAS zugeführt wird, steuert die Motoren 17 und 21 und die Kuppelmagnete 18. An der Auswerteschaltung 23 sind weiterhin zwei Tastschalter 24 und 25 angeschlossen, die das automatische Öffnen und Schließen der Blende 12 bewirken.

Das kreisrunde Feld 26 gibt den durch Röntgenstrahlen durchstrahlten Bereich an und entspricht dem auf dem nicht dargestellten Monitor der Röntgen-Fernsehkette wiedergegebenen Röntgenbild. Als Untersuchungsobjekt 27 wurde als Beispiel ein Kniegelenk einer Untersuchungsperson dargestellt.

In ihrer Ausgangsstellung ist die Blende 12 maximal geöffnet ; alle Lamellen 13 befinden sich außerhalb des Feldes 26. Zu Beginn der Durchleuchtung werden nach Betätigung des Tastschalters 24 die Kuppelmagnete 18 durch die Auswerteschaltung 23 angesteuert, so daß die Zahnstangen 15 in die Zahnwalze 16 eingreifen. Gleichzeitig werden die Motoren 17 derart angesteuert, daß sich die Lamellen 13 aufeinander zu bewegen und sich die Blende 12 langsam schließt. In Abhängigkeit von dem Videosignal BAS werden durch die Auswerteschaltung 23, wie noch beschrieben wird, die einzelnen Kuppelmagnete 18 derart angesteuert, daß sie nacheinander abfallen und somit die Zahnstangen 15 der einzelnen Lamellen 13 nicht mehr mit der sich weiterhin drehenden Zahnwalze 16 in Eingriff stehen, wenn ein bestimmter vorgewählter Helligkeitspegel in dem zur entsprechenden Lamelle 13 gehörenden Videosignal BAS unterschritten wird. Die Steuerung des Motors 21 durch die Auswerteschaltung 23 bewirkt eine Verdrehung des Blendenringes 19, so daß der Schlitz der Blende 12 in bezug auf das Untersuchungsobjekt 27 ausgerichtet wird. Nach Beendigung der Durchleuchtung werden durch Betätigung des Tastschalters 25 über die Auswerteschaltung 23 die Motoren 17 und die Kuppelmagnete 18 derart angesteuert, daß die Blende 12 wieder vollständig geöffnet wird.

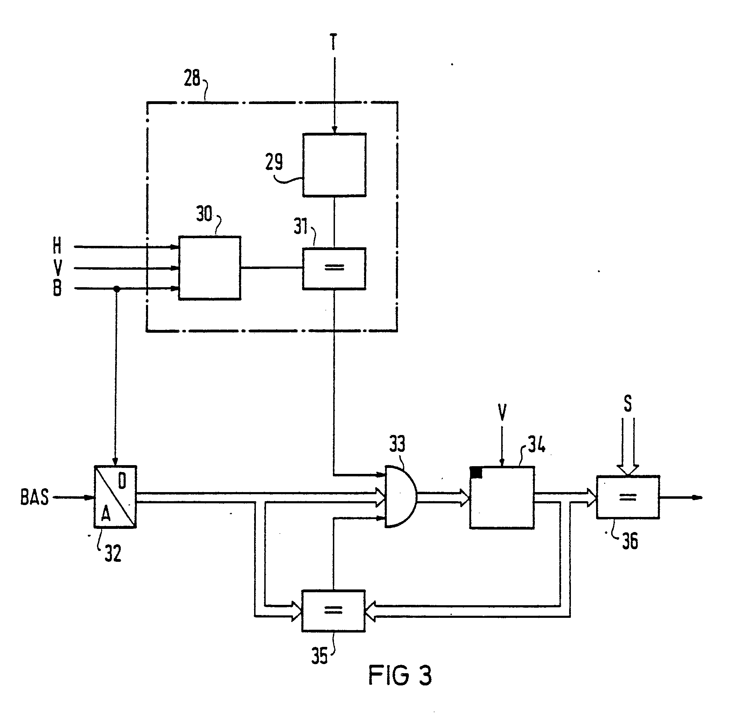

In der Figur 3 ist die Auswerteschaltung 23 dargestellt, die eine Schaltung 28 zur Lagebestimmung der Lamellen 13 innerhalb des Videobildes aufweist. Sie bewirkt eine Zuordnung des aktuellen Videosignales BAS zu den einzelnen Lamellen 13. Sie enthält eine Schaltung 29 zur Festlegung der den einzelnen Lamellen 13 zugeordneten Bereiche, die diese Lamelle 13 vollständig überdecken kann. Dies kann beispielsweise ein Speicher sein, in dem diese Bereiche enthalten sind. Dieser Schaltung 29 wird ein Steuersignal T zugeführt, das die gerade ausgewählte Lamelle 13 kennzeichnet. Ein weiterhin in der Schaltung 28 enthaltener Detektor 30, dem die horizontalen und vertikalen Impulse H und V und der Bildpunkttakt B der Fernsehkette zugeführt werden, dient zur Bestimmung der augenblicklichen Lage des abgetasteten Videosignales BAS. Die Ausgangssignale der Schaltung 29 und des Detektors 30 werden einem ersten Vergleicher 31 zugeführt, der ein Ausgangssignal erzeugt, wenn das Videosignal BAS innerhalb des Bereiches der zur Zeit angesteuerten Lamelle 13 liegt.

Das von der Fernsehkamera kommende Videosignal BAS wird einem Analog/Digital-Wandler (A/D-Wandler 32) zugeführt, der als Anpaßstufe wirkt und dem der Bildpunkttakt zur Digitalisierung zugeführt wird. Das digitale Ausgangssignal des A/D-Wandlers 32 wird einem als Torschaltung wirkenden UND-Glied 33 zugeführt. Die digitalen Ausgangswerte des A/D-Wandlers 32 werden von dem UND-Glied 33 nur durchgelassen, wenn die Schaltung 28 bestimmt hat, daß das ankommende Videosignal BAS zu der zur Zeit angesteuerten Lamelle 13 gehört. Der Ausgang des UND-Gliedes 33 ist mit einem Speicher 34 verbunden, dessen Ausgang auf einen zweiten Vergleicher 35 geführt ist, dessen zweitem Eingang das aktuelle Videosignal des A/D-Wandlers 32 zugeführt wird. Der zweite Vergleicher 35 erzeugt ein Ausgangssignal, wenn der neue Bildpunkt des Videosignales BAS größer ist als der vom Speicher 34 gelieferte alte Bildpunkt, so daß am Ende der Abtastung immer der in dem der angesteuerten Lamelle 13 zugeordneten Bereich des Videosignales enthaltene maximale Amplitudenwert in dem Speicher 34 vorhanden ist. Dadurch arbeiten der Speicher 34, der zweite Vergleicher 35 und das UND-Glied 33 als Spitzenwert-Detektor. Durch die vertikalen Impulse V wird der Speicher 34 nach jedem Fernsehbild gelöscht, so daß erneut der Maximalwert ermittelt werden kann.

Der Ausgang des Speichers 34 wird weiterhin einem dritten Vergleicher 36 zugeführt, an dessen zweitem Eingang ein einstellbarer Schwellenwert S liegt. Das Ausgangssignal des dritten Vergleichers 36 steuert den ausgewählten Kuppelmagneten 18, wenn der in dem Speicher 34 gespeicherte Wert den Schwellenwert S übersteigt.

Im nachfolgenden wird die Funktion der Auswerteschaltung 23 an einem Beispiel näher erläutert. Hierzu wird der Einfachheit halber nur eine der Lamellen 13 betrachtet. Zu Beginn der Durchleuchtung wird von der Schaltung 28 ein H-Signal geliefert, solange sich der vom A/D-Wandler 32 gelieferte Bildpunkt innerhalb des zur angesteuerten Lamelle 13 gehörenden Bereiches im Fernsehbild befindet. Da zu Beginn noch kein Wert im Speicher 34 enthalten war, liefert der zweite Vergleicher 35 ebenfalls ein H-Signal, so daß der erste Bildpunkt in den Speicher 34 eingelesen wird. Dies erfolgt so lange innerhalb des zur angesteuerten Lamelle 13 gehörenden Bereiches, bis der Maximalwert der Bildpunkte in dem Speicher 34 enthalten ist und die folgenden Bildpunkte gleich oder kleiner als der gespeicherte Bildpunkt sind, so daß der zweite Vergleicher 35 ein L-Signal dem UND-Glied 33 zuführt und dieses somit sperrt.

Ist der gespeicherte Bildpunkt größer als der eingestellte Schwellenwert S, so liefert der dritte Vergleicher 36 ein Ausgangssignal, so daß der zur angesteuerten Lamelle 13 gehörende Kuppelmagnet 18 betätigt und die Lamelle 13 durch den Motor 17 bewegt wird, so daß sich die Blende 12 in diesem Bereich schließt. Durch den vertikalen Impuls V wird der Speicher 34 gelöscht, so daß für das nächste Fernsehbild wiederum der Maximalwert in den Speicher eingelesen werden kann. Dies erfolgt so lange, bis sich die Lamelle 13 dem Untersuchungsobjekt 27 genähert hat, so daß von nun an der Maximalwert der Bildpunkte innerhalb des Bereiches der Lamelle 13 geringer wird. Unterschreitet der Maximalwert den dem Vergleicher 36 zugeführten Schwellenwert S, wird der Kuppelmagnet 18 der angesteuerten Lamelle 13 nicht mehr erregt, so daß er abfällt und die Lamelle 13 in dieser halb geschlossenen Stellung verharrt. Anschließend werden die übrigen Lamellen 13 in gleicher Weise angesteuert, so daß sie beispielsweise die in Figur 2 dargestellte Stellung einnehmen.

Anstelle der aufeinanderfolgenden Ansteuerung der Kuppelmagnete 18 und der Lamellen 13 können diese alternierend im Multiplexbetrieb angesteuert werden, wenn für jede der Lamellen 13 wenigstens ein Speicher 34 und eine dritte Vergleichsstufe 36 vorgesehen sind. Durch eine dann erforderliche Multiplexschaltung wird das Ausgangssignal des UND-Gliedes 33 dem entsprechenden Speicher 34 und das Ausgangssignal dieses Speichers 34 dem zweiten Vergleicher 35 und dem jeweiligen dritten Vergleicher 36 zugeführt. Dadurch erfolgt eine nahezu gleichzeitige Verstellung der Lamellen 13, so daß sich die Blende 12 auf ihrer gesamten Breite innerhalb kürzester Zeit so weit als erforderlich schließt.

Der dem dritten Vergleicher 36 zugeführte Schwellenwert S kann beispielsweise kontinuierlich von der Untersuchungsperson eingestellt werden. Es lassen sich aber auch beispielsweise Organe entsprechend darstellen, wenn der Schwellenwert über Drucktasten organabhängig auswählbar ist.

Anstelle der Zahnstange 15 und der Zahnwalze 16 können eine Gummiwalze und eine Reibfläche die Verstellung der Lamellen 13 bewirken. Die Kuppelmagnete 18 können auch für jede Seite der Blende 12 durch einen Kuppelmagneten ersetzt werden, der mechanisch verschoben wird und jede Lamelle 13 einzeln nacheinander mit der Zahnstange 16 kuppelt.

Die Verdrehung des Blendenringes 19 und damit die Ausrichtung des Schlitzes der Blende 12 können zusammen mit der Fernsehkamera gekoppelt sein, so daß die Längsrichtung der Lamellen immer in Zeilenrichtung der Fernsehkette liegt. Sie kann aber auch durch eine ähnliche Auswertung des Fernsehbildes aus dem Videosignal BAS vorgenommen werden.

**Patentansprüche**

1. Röntgendiagnostikeinrichtung mit einer Röntgenröhre, einer zumindest bereichsweise halbtransparenten Blende (12), die einen in seiner Breite veränderbaren, die Strahlung durchlassenden Schlitz freigibt, mit einem Röntgenbildverstärker und einer daran angekoppelten Fernsehkamera zur Erzeugung von Videosignalen, die zur Wiedergabe der Videosignale mit einem Monitor verbunden ist, dadurch gekennzeichnet, daß die Blende (12) aus mehreren einzelnen Lamellen (13) besteht, die zu beiden Seiten des Schlitzes parallel aneinander anliegen und individuell in Abhängigkeit von der Größe des zu betrachtenden Gegenstandes (27) in Längsrichtung verstellbar sind, und daß an der Fernsehkamera eine Auswerteschaltung (23) angeschlossen ist, die ein aus dem Videosignal gewonnenes Steuersignal einer Stellvorrichtung (15 bis 18) für die einzelnen Lamellen (13) zuführt.

2. Röntgendiagnostikeinrichtung nach Anspruch 1, dadurch gekennzeichnet, daß die Längsrichtung der Lamellen (13) senkrecht zur Mittellinie des Schlitzes weist.

3. Röntgendiagnostikeinrichtung nach Anspruch 1, dadurch gekennzeichnet, daß die Längsrichtung der Lamellen (13) mit der Mittellinie des Schlitzes einen Winkel zwischen 20° und 90° bildet.

4. Röntgendiagnostikeinrichtung nach Anspruch 1, dadurch gekennzeichnet, daß für jede Seite der Blende (12) ein Motor (17), der eine Walze (16) antreibt, und wenigstens ein Kuppelmagnet (18) vorgesehen sind, die von der Auswerteschaltung (23) gesteuert werden, und daß die Lamellen (13) durch den Kuppelmagneten (18) mit der Walze (16) kuppelbar sind.

5. Röntgendiagnostikeinrichtung nach Anspruch 4, dadurch gekennzeichnet, daß ein Kuppelmagnet (18) vorgesehen ist, der mechanisch verschoben wird und die Lamellen (13) einzeln mit der Walze (16) des Motors (17) kuppelt.

6. Röntgendiagnostikeinrichtung, bei der die Blende (12) auf einem drehbaren Blendenring (19) angeordnet ist, nach Anspruch 1, dadurch gekennzeichnet, daß die Fernsehkamera und die Blende in ihrer Drehung miteinander derart gekoppelt sind, daß die Längsrichtung der Lamellen (13) immer in Zeilenrichtung der Fernsehkette liegt.

7. Röntgendiagnostikeinrichtung nach Anspruch 1, dadurch gekennzeichnet, daß die Auswerteschaltung (23) eine Schaltung (28) zur Lagebestimmung der Lamellen (13) innerhalb des Videobildes aufweist, der die Taktimpulse (B, H, V) der Fernsehkamera und der Steuersignale (T) zugeführt werden, die die jeweils angesteuerte Lamelle (13) kennzeichnen, daß das Videosignal (BAS) einer Anpaßstufe (32) zugeführt ist, daß die Anpaßstufe (32) und die Schaltung (28) mit einer Torschaltung (33) verbunden sind, an der ein Spitzenwert-Detektor (34, 35) angeschlossen ist, daß das Ausgangssignal des Detektors (34, 35) einer Vergleichsstufe (36) zugeführt wird, die es mit einem einstellbaren Schwellenwert (S) vergleicht, und daß die Vergleichsstufe (36) die Kuppelmagnete (18) steuert.

## Claims

1. X-ray diagnostic apparatus having : an X-ray tube ; a diaphragm (12) which is semi-transparent at least in zones and which leaves free a slot which is variable in its width and which lets through the radiation ; an X-ray image intensifier ; and a video camera coupled to it to generate video signals, which video camera is connected to a monitor in order to reproduce the video signals, characterised in that the diaphragm (12) consists of several individual laminae (13), abutting one another parallel to both sides of the slot and individually adjustable in the longitudinal direction in dependence on the size of the subject of observation (27), and in that there is connected to the video camera an evaluating circuit (23) which supplies a control signal obtained from the video signal to an adjusting device (15 to 18) for the individual laminae (13).

2. X-ray diagnostic apparatus according to claim 1, characterised in that the longitudinal direction of the laminae (13) is directed perpendicularly to the centre line of the slot.

3. X-ray diagnostic apparatus according to claim 1, characterised in that the longitudinal direction of the laminae (13) forms an angle of between 20° and 90° with the centre line of the slot.

4. X-ray diagnostic apparatus according to claim 1, characterised in that for each side of the diaphragm (12) there is provided a respective motor (17), which drives a roller (16), and at least one coupling magnet (18), which motor and magnet are controlled by the evaluating circuit (23), and in that the laminae (13) may be coupled with the roller (16) by means of the coupling magnet (18).

5. X-ray diagnostic apparatus according to claim 4, characterised in that there is provided a coupling magnet (18) which is mechanically displaceable and which couples the laminae (13) individually with the roller (16) of the motor (17).

6. X-ray diagnostic apparatus, in which the diaphragm (12) is arranged on a rotatable diaphragm ring (19), according to claim 1, characterised in that the video camera and the diaphragm are coupled in their rotation with one another such that the longitudinal direction of the laminae (13) always lies in the line direction of the video transmission.

7. X-ray diagnostic apparatus according to claim 1, characterised in that the evaluating circuit (23) has a circuit (28) for the determination of the position of the laminae (13) within the video image and to which are supplied the timing pulses (B, H, V) of the video camera and of the control signals (T) which designate the particular lamina (13) being controlled at the time, in that the video signal (BAS) is supplied to a matching stage (32), in that the matching stage (32) and the circuit (28) are connected to a gate circuit (33) to which is connected a peak value detector (34, 35), in that the output signal of the detector (34, 35) is supplied to a comparison stage (36) which compares it with an adjustable threshold value (S), and in that the comparison stage (36) controls the coupling magnets (18).

## Revendications

1. Appareil de radiodiagnostic comportant un tube à rayons X, un diaphragme (12) semi-transparent au moins par zones, qui dégage une fente, dont la largeur est variable et qui laisse passer le rayonnement, un amplificateur de brillance radiologique et une caméra de télévision couplée à cet amplificateur, servant à produire des signaux vidéo et reliée à un moniteur pour la reproduction des signaux vidéo, caractérisé par le fait que le diaphragme (12) est constitué par plusieurs lamelles individuelles (13), qui s'appliquent parallèlement les unes contre les autres des deux côtés de la fente et peuvent être déplacées individuellement dans la direction longitudinale en fonction de la taille de l'objet (27) à observer, et qu'à la caméra de télévision est raccordé un circuit d'évaluation (23) qui envoie un signal de commande, obtenu à partir du signal vidéo, à un dispositif de réglage (15 à 18) prévu pour les différentes lamelles (13).

2. Appareil de radiodiagnostic suivant la revendication 1, caractérisé par le fait que la direction longitudinale des lamelles (13) est perpendiculaire à l'axe médian de la fente.

3. Appareil de radiodiagnostic suivant la revendication 1, caractérisé par le fait que la direction longitudinale des lamelles (13) fait avec l'axe médian de la fente un angle compris entre 20° et 90°.

4. Appareil de radiodiagnostic suivant la revendication 1, caractérisé par le fait que pour chaque côté du diaphragme (12), il est prévu un moteur (17), qui entraîne un cylindre (16), et au moins un aimant d'accouplement (18), le moteur et l'aimant étant commandés par le circuit d'évaluation (23), et que les lamelles (13) peuvent être accouplées au cylindre (16) au moyen des aimants d'accouplement (18).

5. Appareil de radiodiagnostic suivant la revendication 4, caractérisé par le fait qu'il est prévu un aimant d'accouplement (18) qui est déplacé mécaniquement et accouple individuellement les lamelles (13) au cylindre (16) du moteur (17).

6. Appareil de radiodiagnostic, dans lequel le diaphragme (12) est monté sur une bague rotative (19) de support du diaphragme, suivant la revendication 1, caractérisé par le fait que la caméra de télévision et le diaphragme sont couplés entre eux, pour leur rotation, de manière que la direction longitudinale des lamelles (13) s'étendent toujours dans la direction des lignes de la chaîne de télévision.

7. Appareil de radiodiagnostic suivant la revendication 1, caractérisé par le fait que le circuit d'évaluation (23) comporte un circuit (28) qui sert à déterminer la position des lamelles (13) dans l'image vidéo et auquel sont envoyées les impulsions de cadence (B, H, V) de la caméra de télévision et des signaux de commande (T), qui caractérisent les lamelles (13) respectivement commandées, que le signal vidéo (BAS) est envoyé à un étage d'adaptation (32), que l'étage d'adaptation (32) et le circuit (28) sont reliés à un circuit de porte (33), auquel est raccordé un détecteur de valeur maximale (34, 35), que le signal de sortie du détecteur (34, 35) est envoyé à un étage comparateur (36), qui compare ce signal à une valeur de seuil réglable (S), et que l'étage comparateur (36) commande les aimants d'accouplement (18).

FIG 1

FIG 2

FIG 3